# EUROPEAN PATENT APPLICATION

(11) **EP 1 407 789 A1**
(43) Date of publication of application: **14.04.2004**
(21) Application number: 03380197.8
(22) Date of filing: 03.09.2003
(51) Int. Cl.: A61L 9/03

(54) **Evaporator device of volatile substances**

(30) Priority: 09.10.2002 ES 200202413 U
(71) Applicant: BOBINAJES NUGAR, S.L., 08191 Rubi (ES)
(72) Inventor: Garcia Nunez, Ginés Jorge, c/o Pol.Ind.Can Roses, a Sabadell km.13, 08191 RUBI (ES)
(74) Representative: SUGRANES - VERDONCES - FERREGÜELA

(57) **Abstract**

Evaporator device (1) of volatile substances for large areas according to claim 1, characterised in that it comprises a flat base (8), provided with lateral walls (14) integral to the whole perimeter of the base, and an extractible top cover (9) that contains the electrical resistor (4). Said device is fitted with a casing (6) intended for housing at least one receptacle, which consists of a cavity (7) delimited by at least one flat base (8) where the receptacle rests, a top cover (9) that contains the electrical resistor, fixing means (10) of the device to the wall and a fan (11) coupled into a notch (12) made in said base.

## Description

### Technical sector of the invention

The present invention relates to an evaporator device of volatile substances for large areas, particularly applicable to receptacles containing an impregnated wick of compounds to be evaporated by heating; said device comprising an electrical resistor which encircles the wick and a plug-in connection to the mains.

### Background to the invention

Air fresheners, or evaporators of relevant substances, are well-known and widely used, whether perfumes or insect repellents, which are used by electricity and which, by means of heating a resistor, allow for the diffusion of the desired volatile substance. In the domestic sphere, small receptacles are used, which can usually be easily refilled. When the aim is to create an ambience, or volatise relevant substances in large areas, the problem of current electrical devices not being large enough arises and many units must be placed in order to achieve the desired effect. For many years, liquid atomisation devices, controlled by a timer, have been used, but said devices entail the disadvantage of not allowing for a steady and constant vaporisation in time. Large receptacles can also be used, without electricity and with liquids which impregnate a wick, but many units are necessary and their diffusion is not constant either. The possibility of using larger receptacles connected to the mains present a problem in that they must not exceed the weight which, for safety reasons, the power point is able to take. Utility model U no. 9501706, discloses an air freshener that would solve the problem of excess weight on the power point, being a relatively large-sized air freshener with a wick encircled by an resistor connected to a wire, but the bottle has to be placed on the floor or on a table surface, as, if knocked, its content can be very easily spilt. Also, utility model no. 1049393 puts forward a solution to the problem of vaporizing substances in large areas, being based on the cumulative effect of several evaporators, in this case by means of a container which makes it possible to connect several of them to the mains by using a common electrical strip. The object of the present invention represents an alternative to the existing inventions and a solution to the problems set out earlier.

### Explanation of the invention

The evaporator device of volatile substances for large areas object of the present invention, is characterised in that it comprises a casing intended to contain at least one receptacle, said casing consisting of a cavity, delimited by at least one flat base where the aforementioned receptacle rests, a top cover containing an electrical resistor, fixing means of the device to the wall and a fan coupled into a notch made to said base, all of which is arranged so that the weight of the receptacle, the resistor and the fan, is supported by the casing structure.

According to another characteristic of the invention, the casing comprises a flat base provided with lateral walls which are integral to its whole perimeter, and an extractible top cover that contains the electrical resistor.

### Brief description of the drawings

In the attached drawings, a form of embodiment of the object of the present invention is illustrated by way of non-limiting example, affording a better understanding thereof. In said drawings:
- Fig. 1: shows a diagrammatic perspective view of the evaporator device of volatile substances; and
- Fig. 2: is also a diagrammatic perspective view of the preferred form of the device of the invention.

### Detailed description of the drawings

As can be appreciated in the attached drawings, the evaporator device 1 of volatile substances for large areas object of the present invention, comprises a casing 6 designed to receive receptacles 2 containing liquids with relevant substances to be evaporated by heating a wick integrated within the receptacle 2, said wick 3 being impregnated with the aforementioned liquid.

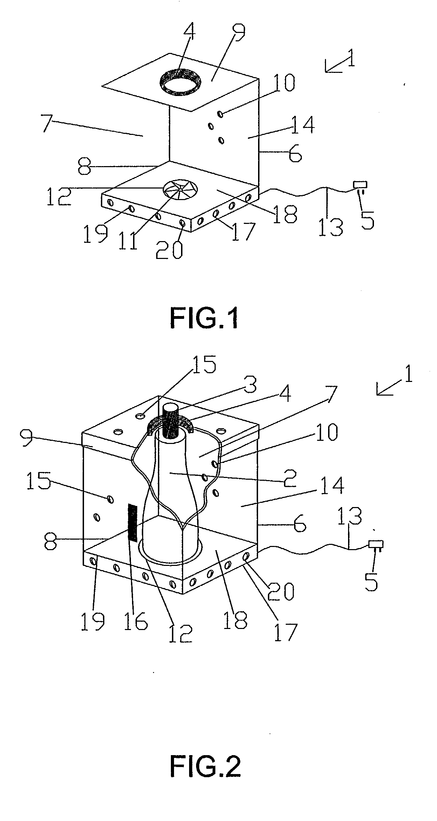

As exemplified in Fig. 1, said casing 6 consists of a cavity 7 determined by a flat base 8; a top cover 9 with an electrical resistor contained therein; fixing means 10, which in the case represented are orifices made in a lateral wall 14 of the device 1; and a fan 11 fitted into a cavity 12 made for that purpose in the base 8. The device 1 connects to the mains by a wire 13, thus easing the situating of the aforementioned device 1 in the desired location. An evaporator device 1 with said features is easily fixed onto a wall and at a suitable height. Also, in order that the device 1 may be situated on the floor, it has been envisaged that the flat base 8 is comprised of a tray 17, which contains and protects the mechanism from the fan in its interior and which is arranged in such a way that its covered face 18 supports the receptacle 2, the walls 21 of the tray 17 comprising several orifices 20 in order to allow the intake and impulsion of the air by the fan 11.

Fig. 2 corresponds to the preferred representation of the evaporator device 1 object of the invention wherein it can be appreciated that the casing 6 comprises opaque and transparent lateral walls 14, integral to the whole perimeter of the flat base 8, on which the receptacle 2 rests with the wick 3 encircled or circumscribed by the electrical resistor 4, housed in an extractible top cover 9. A notch, as if it were a vision slit, has been made in one of the lateral walls 14, very advantageous for checking the liquid level without having to dismantle the device 1 in the event of there being opaque lateral walls 14. Furthermore, the casing surface 6 comprises a plurality of orifices 15 with a view to easing the diffusion of the evaporated volatile substances.

An evaporator device 1 like the one described, allows for the homogeneous and constant evaporation of a maximum of 2 grams of volatile substance per hour and has a consumption of around 2 to 3 w/h, being particularly applicable for the evaporation of volatile substances in large areas, such as for example: commercial establishments, farms, veterinary consulting rooms, greenhouses, sanitary areas, public toilets, restaurants, industries, etc. Specifically, in the case of its being used on farms, it has the advantage of being able to vaporise substances against pests or pathogenic germs which should otherwise be applied directly to the animals.

Having sufficiently described the nature of the present invention, as well as the way to put it into practice, it is herein stated that all that which does not alter, change or modify its main principle, is subject to variations in detail.

## Claims

1. Evaporator device (1) of volatile substances for large areas, applicable to receptacles (2) that contain a wick (3) impregnated with compounds to be evaporated by heating, said device comprising an electrical resistor (4) which encircles the wick and a connection for the electrical supply (5), **characterised in that** it is fitted with a casing (6) intended for housing at least one receptacle, which consists of a cavity (7) delimited by at least one flat base (8) where the receptacle rests, a top cover (9) that contains the electrical resistor, fixing means (10) of the device to the wall and a fan (11) coupled into a notch (12) made in said base, all in such a way that the weight of the receptacle, the resistor and the fan is supported by the casing structure.

2. Evaporator device (1) of volatile substances for large areas according to claim 1, **characterised in that** it comprises a flat base (8), provided with lateral walls (14) integral to the whole perimeter of the base, and an extractible top cover (9) that contains the electrical resistor (4).
